# EUROPÄISCHE PATENTSCHRIFT

(11) **EP 1 444 956 B1**
(45) Veröffentlichungstag und Bekanntmachung des Hinweises auf die Patenterteilung: **13.06.2007**
(21) Anmeldenummer: 04002342.6
(22) Anmeldetag: 03.02.2004
(51) Int. Cl.: A61B 17/15

(54) **Vorrichtung zur Ausbildung eines Knochenschnittes**
Positioning device for performing a cut in bone
Dispositif de positionnement pour exécuter une coupure d'os

(30) Priorität: 07.02.2003 DE 10305126; 03.04.2003 DE 10315259
(43) Veröffentlichungstag der Anmeldung: 11.08.2004
(73) Patentinhaber: Plus Orthopedics AG, 6343 Rotkreuz (CH)
(72) Erfinder: Brack, Renè, 6330 Cham (CH); Rickenbacher, Pius, 6312 Steinhausen (CH); Kilchenmann, Thomas, 8810 Horgen (CH)
(74) Vertreter: Popp, Eugen

(56) Entgegenhaltungen:
- FR-A- 2 790 381
- US-A- 5 662 656
- US-A- 5 681 316
- US-A1- 2002 198 531

## Beschreibung

Die Erfindung betrifft eine Vorrichtung zur Ausbildung eines Knochenschnitts, insbesondere am proximalen Ende einer Tibia, mit einer dem Knochen zuordenbaren Anlagefläche für ein Schneid- oder Sägewerkzeug, eine Ausrichtlehre und/oder ein Messinstrument.

Derartige Vorrichtungen sind allgemein bekannt. Nachteilig ist jedoch die richtige Justierung am Knochen so, dass ein Schnitt in vorbestimmter Ebene durchgeführt werden kann. Die herkömmlichen Vorrichtungen erlauben keine Nachjustierung der Anlagefläche für ein Schneid- oder Sägewerkzeug, eine Ausrichtlehre und/oder ein Messinstrument. Es muß entweder die Vorrichtung insgesamt neu am Knochen gesetzt werden; oder es müssen am Werkzeug selbst Vorkehrungen getroffen werden, um eine Nachjustierung zu ermöglichen. Insofern ist der Operateur auf ein entsprechend aufwendiges Werkzeug angewiesen. Die andere Alternative der Neuausrichtung der gesamten Vorrichtung am Knochen ist ebenfalls problematisch.

So zeigt die US-A-5681316 eine Führungsvorrichtung für eine Tibiaresektion, die einen Führungsblock mit Bohrungen aufweist, mit Hilfe derer die Tibia an dem Führungsblock befestigt wird. Ferner weist der Führungsblock einen Schlitz auf, der geeignet ist, ein Sägeblatt aufzunehmen, das für die Resektion der Tibia verwendet wird. Der Führungsschlitz dient als Anlagefläche bzw. als Führung für das Schneid- bzw. Sägewerkzeug, das bei der Tibiaresektion verwendet wird. Bei dem dort offenbarten Gegenstand liegt folglich die Tibia einerseits fest an dem Führungsblock an und wird andererseits mittels eines Sägeblattes geschnitten, das seinerseits durch den im Führungsblock vorgesehenen Schlitz geführt wird. Aus diesem Grund ist die Schneidrichtung und der Schneidwinkel, mit dem die Tibia mittels des Sägeblatts geschnitten wird, sehr eng mit der Befestigung der Tibia an dem Führungsblock assoziiert, so daß die Einstell- und Justiermöglichkeiten der dortigen Tibiaresektionsvorrichtung, insbesondere in bezug auf Schneidrichtung und Schneidwinkel, unzureichend sind.

Der vorliegenden Erfindung liegt daher die Aufgabe zugrunde, eine Vorrichtung der eingangs genannten Art zu schaffen, die eine uneingeschränkte Justierung der Anlagefläche für ein Schneid- oder Sägewerkzeug, eine Ausrichtlehre und/oder ein Messinstrument erlaubt, nachdem die Vorrichtung am Knochen fixiert worden ist.

Diese Aufgabe wird durch die kennzeichnenden Merkmale des Anspruches 1 gelöst, wobei bevorzugte konstruktive Details in den Unteransprüchen beschrieben sind.

Der Kern der vorliegenden Erfindung liegt also an der universalgelenkigen Lagerung eines Justierelements, welches Teil der Gesamtvorrichtung ist und das die erwähnte Anlagefläche umfaßt. Die universalgelenkige Lagerung erlaubt eine Justierung um zwei zueinander senkrechte Achsen, insbesondere Horizontal-Achsen, vorzugsweise um eine sich posterior/anterior erstreckende Achse einerseits und um eine sich medial/lateral erstreckende Achse andererseits.

Zusätzlich kann noch vorgesehen sein eine Justiermöglichkeit um eine Achse etwa parallel zur Knochenlängsachse, zum Beispiel um eine Achse parallel zur Tibialängsachse, um z.B. einen unerwünschten Versatz einer Schnittlehre beim Setzen am Knochen ausgleichen zu können.

Die letztgenannte Vorrichtung zeichnet sich also durch eine Justiermöglichkeit um drei zueinander senkrecht stehende Achsen aus

Von besonderer konstruktiver Bedeutung ist die Maßnahme nach Anspruch 5, wonach das Justierelement samt erwähnter Anlagefläche über einen Zwischenlagerbock am Lagerbock gehalten ist, wobei der Zwischenlagerbock am Lagerbock um die erste Achse und das Justierelement am Zwischenlagerbock um die zweite Achse schwenkbar gelagert ist. Die Einstellung erfolgt jeweils durch zugeordnete Justierschrauben. Diese Justierschrauben können entweder gegen die Wirkung eines elastischen Elements, insbesondere gegen die Wirkung einer Schraubendruckfeder verstellbar sein, oder zum Zwecke der Verstellung rein formschlüssig zwischen den relativ zueinander verschwenkbaren Teilen auf diese einwirken, zum Beispiel wie in Anspruch 8 angegeben.

Die Schwenklage der einzelnen Teile relativ zueinander wird vorzugsweise durch entsprechende Markierungen visuell angezeigt. Diese Markierungen können auch Skalen umfassen.

Der Schwenkbereich um die jeweiligen Achsen beträgt zwischen etwa ± 2,0° und ± 6,0°, vorzugsweise etwa ± 3° und ± 4°.

Nachstehend wird eine bevorzugte Ausführungsform einer erfindungsgemäßen Vorrichtung anhand der beigefügten Zeichnung näher erläutert. Diese zeigt in:
- Fig. 1: eine perspektivische Ansicht einer Vorrichtung zur Ausbildung eines Knochenschnitts von der dem Knochen abgewandten Seite;
- Fig. 2: eine perspektivische Ansicht der Vorrichtung gemäß Fig. 1 von der Knochenseite;
- Fig. 3: eine Ansicht entsprechend Fig. 1, bei der sich die Anlagefläche bzw. das entsprechende Justierelement in Außer-Nullstellung befindet;
- Fig. 4: die Vorrichtung gemäß Fig. 1 oder 2 in Stirnansicht entsprechend Pfeil IV in Fig. 2;
- Fig. 5: die Vorrichtung gemäß Fig. 1 und 2 in Stirnansicht von der gegenüberliegenden Seite bzw. in Richtung des Pfeils V in Fig. 1;
- Fig. 6: die Vorrichtung gemäß Fig. 3 in Stirnansicht entsprechend Fig. 4 unter Darstellung eines sog. dorsalen slope's der Anlagefläche;
- Fig. 7: die Vorrichtung gemäß den Fig. 1 bis 3 in Frontansicht von der knochenabgewandten Seite unter Darstellung einiger Details innerhalb der Vorrichtung;
- Fig. 8: die Vorrichtung in Ansicht entsprechend Fig. 7, wobei die Anlagefläche bzw. das dazugehörige Justierelement relativ zu einem Lagerbock, der am Knochen fixierbar ist, verschwenkt ist (in Richtung varus oder valgus); und
- Fig. 9: die Vorrichtung gemäß den Figuren 1 bis 3 in Draufsicht.

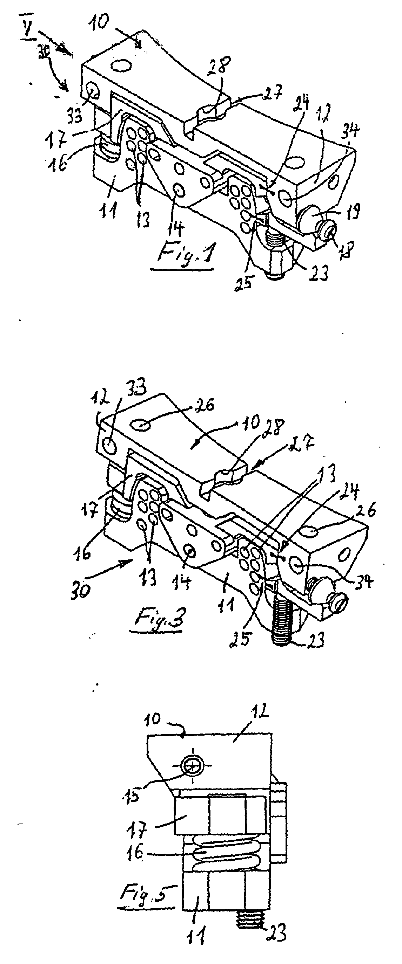

In den Figuren 1 bis 9 ist eine Vorrichtung 30 zur Ausbildung eines Knochenschnitts, zum Beispiel am proximalen Ende einer Tibia, mit einer dem Knochen (nicht dargestellt) zuordenbaren Anlagefläche 10 für ein Schneid- oder Sägewerkzeug, eine Ausrichtlehre und/oder ein Messinstrument dargestellt. Zur Fixierung eines Messinstruments sind an der Anlagefläche 10, d.h. der Oberseite der Vorrichtung 30 zwei Gewindebohrungen 26 vorgesehen.

Die Befestigung einer Ausrichtlehre erfolgt innerhalb eines in der Anlagefläche 10 ausgebildeten zentralen Einschnitts 27, der eine mittig angeordnete Zentralbohrung 28 umfaßt.

Die Anlagefläche 10 ist Teil eines Justierelements 12, das an einem am (nicht dargestellten) Knochen befestigbaren Lagerbock universalgelenkig gelagert ist. Zur Befestigung des Lagerbocks 11 am Knochen weist dieser mehrere Durchgangsbohrungen 13 zur Aufnahme von Knochenschrauben oder Knochen-Fixationsstiften auf, wobei einige Bohrungen auch schräg ausgerichtet sein können, wie die Bohrungen 29 in Figur 7.

Vorzugsweise wird der Lagerbock 11 samt Justierelement 12 so am Knochen fixiert, dass das Justierelement 12 um eine sich senkrecht zum Knochen, insbesondere sich posterior/anterior erstreckende erste Achse 14 sowie um eine sich senkrecht sowohl zu dieser ersten Achse 14 als auch zur Knochenlängsachse, insbesondere medial/lateral erstreckende zweite Achse 15 verschwenkbar gelagert ist. Um diese Universalgelenkigkeit zu ermöglichen, ist das Justierelement 12 über einen Zwischenlagerbock 17 am Lagerbock 11 gehalten, wobei der Zwischenlagerbock 17 am Lagerbock 11 um die erste Achse 14 und das Justierelement 12 am Zwischenlagerbock 17 um die zweite Achse 15 schwenkbar gelagert ist. Das Justierelement 12 samt Anlagefläche 10 ist nach Art einer Wippe, und zwar über dem Zwischenlagerbock 17 am Lagerbock 11 schwenkbar gelagert und mittels einer am Lagerbock 11 einerseits und am Zwischenlagerbock 17 andererseits abgestützten ersten Justierschraube 23 um die erste Achse 14 verschwenkbar, wobei die Verschwenkung gegen die Wirkung eines elastischen Elements, nämlich einer Schraubendruckfeder 16 erfolgt. Die Justierschraube 23 wirkt also relativ zur ersten Schwenkachse 14 an einer Seite des Zwischenlagerbocks 17, während die Schraubendruckfeder 16 an der gegenüberliegenden Seite wirksam ist. Die Schraubendruckfeder 16 dient gleichzeitig zur Blockierung der Gewindeverbindung zwischen Justierschraube 23 und Lagerbock 11. Die Schraubendruckfeder 16 ist innerhalb topfförmiger Ausnehmungen an der Oberseite des Lagerbocks 11 einerseits und an der Unterseite des Zwischenlagerbocks 17 andererseits platziert. Die erwähnten Ausnehmungen sind in Figur 7 mit der Bezugsziffer 31 und 32 gekennzeichnet.

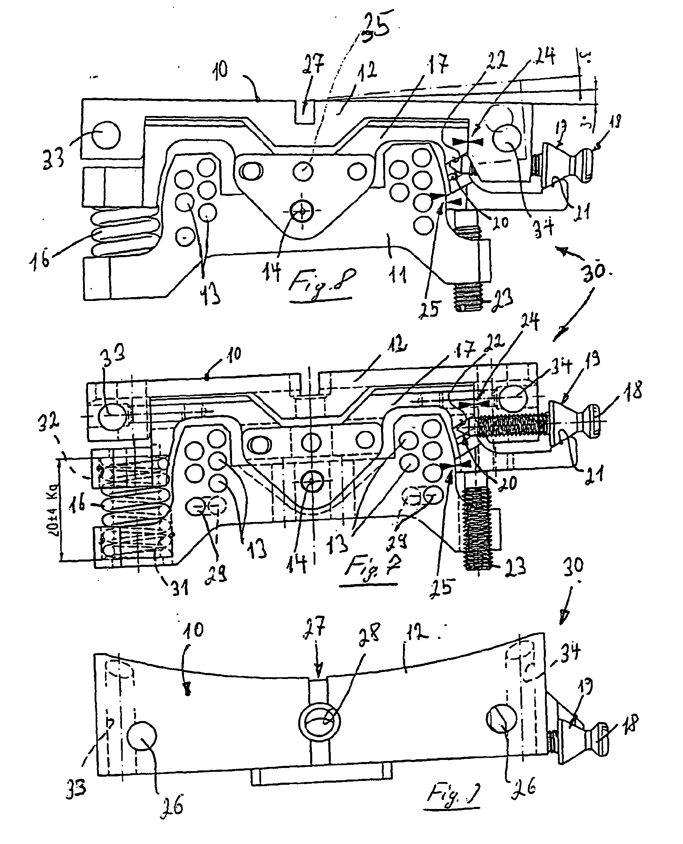

Die Schwenkbewegung des Justierelements 12 um die erste Schwenkachse 14 im Lagerbock 11 erfolgt also unter gleichzeitigem Mitverschwenken des Zwischenlagers 17. Es wird diesbezüglich insbesondere auf Figur 8 im Vergleich zu Figur 7 verwiesen. Figur 8 zeigt auch den Schwenkbereich von ± 3° ausgehend von einer Horizontallage. Sofern sich die Schwenkachse 14 in Richtung posterior/anterior erstreckt, kann somit eine vorbestimmte varus/valgus-Stellung der Anlagefläche 10 eingestellt werden. Die gewünschte Stellung wird visuell durch eine an der knochenabgewandten Seite der Vorrichtung angeordnete Markierung 25 angezeigt. Diese Markierung kann noch mit einer Winkelgrad-Skala versehen sein. Die Anzeige der Schwenklage des Justierelements 12 kann auch durch einen sich in Tibialängsachse erstreckenden Stab erfolgen, der z.B. in einer der in Fig. 8 oberhalb der Schwenkachse 14 angeordneten Bohrungen 35 befestigt ist.

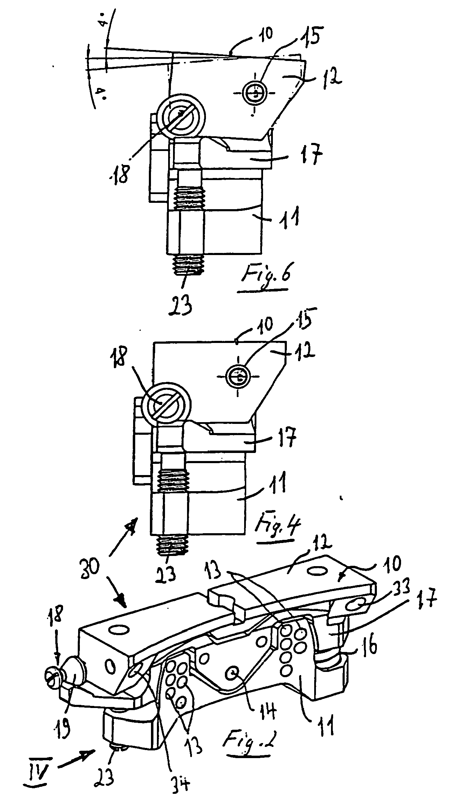

Das Justierelement 12 ist schließlich noch am Zwischenlagerbock 17 um die zweite Schwenkachse 15 verschwenkbar, wobei die Schwenklage durch eine zweite Justierschraube 18 eingestellt wird. Diese zweite Justierschraube 18 ist in besonderer Weise ausgebildet. Sie umfaßt zwei gegenseitig zueinander geneigte Kegelflächen bzw. Kegelflächenabschnitte 19, 20, die mit komplementären Schrägflächen 21, 22 am Zwischenlagerbock 17 derart korrespondieren, dass durch Verstellen der Justierschraube 18 das Justierelement 12 um die zweite Achse 15 entweder nach dorsal oder nach ventral verschwenkt und in dieser Lage gehalten wird. Der entsprechende Schwenkwinkelbereich von ± 4° ist in Figur 6 dargestellt. Die Figuren 4 und 6 lassen im übrigen auch noch erkennen, dass die Justierschraube 18 seitlich zur Schwenkachse 15 versetzt ist, und zwar in Richtung vom Knochen weg. Dadurch lässt sich mittels der Justierschraube 18 die gewünschte Verschwenkung nach dorsal/ventral um die Schwenkachse 15 erreichen. Wie insbesondere die Figuren 7 und 8 sehr gut erkennen lassen, erfolgt die Schwenkbewegung und Fixierung des Justierelements 12 um die zweite Schwenkachse 15 ausschließlich durch formschlüssige Zusammenwirkung zwischen der am Justierelement 12 angeordneten Justierschraube 18 und dem Zwischenlagerbock 17. Grundsätzlich ist natürlich auch eine Justierung entsprechend der beschriebenen Justierung des Zwischenlagerbocks 17 relativ zum Lagerbock 11 denkbar.

Auch der zweiten Schwenkachse 15 ist eine Markierung bzw. Skalierung 24 zugeordnet, um den Schwenkwinkel nach dorsal oder ventral visuell an der dem Knochen abgewandten Seite der Vorrichtung 30 erkennen zu können.

Das Justierelement 12 umfaßt auch noch zwei Bohrungen parallel zu den Durchgangsbohrungen 13, und zwar an den beiden äußeren Seiten des Justierelements. Diese Bohrungen, die mit der Bezugsziffer 33, 34 versehen sind, dienen ebenfalls zur Fixierung einer Schnittlehre, eines Messinstruments, der Halterung eines Schneid- oder Sägewerkzeuges od. dgl.. Die Bohrungen 33, 34 können ebenfalls mit einem Innengewinde versehen sein.

Sämtliche in den Anmeldungsunterlagen offenbarten Merkmale werden als erfindungswesentlich beansprucht, soweit sie einzeln oder in Kombination gegenüber dem Stand der Technik neu sind.

### Bezugszeichen

- 10: Anlagefläche
- 11: Lagerbock
- 12: Justierelement
- 13: Durchgangsbohrung
- 14: erste Schwenkachse
- 15: zweite Schwenkachse
- 16: Schraubendruckfeder
- 17: Zwischenlagerbock
- 18: Zweite Justierschraube
- 19: Kegelfläche
- 20: Kegelfläche
- 21: Schrägfläche
- 22: Schrägfläche
- 23: erste Justierschraube
- 24: Markierung
- 25: Markierung
- 26: Gewindebohrung
- 27: Einschnitt
- 28: Zentralbohrung
- 29: Schrägbohrung
- 30: Vorrichtung zur Ausbildung eines Knochenschnitts
- 31: Ausnehmung
- 32: Ausnehmung
- 33: Bohrung
- 34: Bohrung
- 35: Bohrung

## Patentansprüche

1. Vorrichtung zur Ausbildung eines Knochenschnitts, zum Beispiel am proximalen Ende einer Tibia, mit einer dem Knochen zuordenbaren Anlagefläche (10) für ein Schneid- oder Sägewerkzeug, eine Ausrichtlehre und/oder ein Messinstrument,
**dadurch gekennzeichnet, dass**
die Anlagefläche (10) Teil eines Justierelements (12) ist, das über einen Zwischenlagerbock (17) an einem am Knochen befestigbaren Lagerbock (11) universalgelenkig gehalten ist, wobei der Zwischenlagerbock (17) am Lagerbock (11) um eine erste Achse (14) und das Justierelement (12) am Zwischenlagerbock (17) um eine zweite Achse (15) schwenkbar gelagert ist, wobei weiterhin das Justierelement (12) nach Art einer Wippe, insbesondere über den Zwischenlagerbock (17), am Lagerbock (11) schwenkbar gelagert und mittels einer am Lagerbock (11) einerseits und am Justierelement (12) oder einem Zwischenlagerbock (17) andererseits abgestützten ersten Justierschraube (23) um eine erste Achse (14) verschwenkbar ist, vorzugsweise entgegen der Wirkung eines elastischen Elements, insbesondere einer Schraubendruckfeder (16).

2. Vorrichtung nach Anspruch 1,
**dadurch gekennzeichnet, dass**
die Anlagefläche (10) bzw. das zugeordnete Justierelement (12) um die Achse etwa parallel zur Knochenlängsachse verschwenkbar ist.

3. Vorrichtung nach Anspruch 1 oder 2,
**dadurch gekennzeichnet, dass**
der Lagerbock (11) mehrere Durchgangsbohrungen (13, 29) für Knochenschrauben oder Knochen-Fixationsstifte aufweist.

4. Vorrichtung nach einem der Ansprüche 1 bis 3,
**dadurch gekennzeichnet, dass**
der Lagerbock (11) samt Justierelement (12) so am Knochen fixierbar ist, dass das Justierelement (12) um eine sich senkrecht zum Knochen, insbesondere sich posterior/anterior erstreckende erste Achse (14) sowie um eine sich senkrecht sowohl zu dieser ersten Achse (14) als auch zur Knochenlängsachse, insbesondere medial/lateral erstreckende zweite Achse (15) verschwenkbar gelagert ist.

5. Vorrichtung nach einem der Ansprüche 1 bis 4,
**dadurch gekennzeichnet, dass**
das Justierelement (12) über den Zwischenlagerbock (17) am Lagerbock (11) gehalten ist, wobei der Zwischenlagerbock (17) am Lagerbock (11) um eine, insbesondere in die erste Achse (14) und das Justierelement (12) am Zwischenlagerbock (17) um eine andere, insbesondere die zweite, sich senkrecht zur ersten Achse (14) erstreckende Achse (15) schwenkbar gelagert ist.

6. Vorrichtung nach einem der Ansprüche 1 bis 5,
**dadurch gekennzeichnet, dass**
das Justierelement (12) samt Anlagefläche (10) am Zwischenlagerbock (17) durch eine zweite Justierschraube (18) um die zweite Achse (15) verschwenk- und einstellbar ist.

7. Vorrichtung nach Anspruch 6,
**dadurch gekennzeichnet, dass**
die zweite Justierschraube (18) zwei gegenseitig zueinander geneigte Kegelflächen (19, 20) aufweist, die mit komplementären Schrägflächen (21, 22) am Zwischenlagerbock (17) derart korrespondieren, dass durch Verstellen der Justierschraube (18) das Justierelement (12) um die zweite Achse (15) entweder nach dorsal oder nach ventral verschwenkt und in dieser Lage gehalten wird.

8. Vorrichtung nach einem der Ansprüche 1 bis 7,
**dadurch gekennzeichnet, dass**
am Justierelement (12) und Zwischenlagerbock (17) einerseits und/oder am Zwischenlagerbock (17) und Lagerbock (11) andererseits jeweils miteinander korrespondierende Markierungen (24, 25) vorgesehen sind.

## Claims

1. Device for the formation of a bone cut, for example, at the proximal end of a tibia, with a retaining surface (10), assigned to the bone, for a cutting tool or saw, an alignment guide and/or a measuring instrument,
**characterised in that**
the retaining surface (10) is a component of an adjustment element (12), which is held in a universally-articulated manner via an intermediate mounting block (17) on a mounting block (11), which can be attached to the bone, wherein the intermediate mounting block (17) is mounted in such a manner that it can be rotated about a first axis (14), and the adjustment element (12) is mounted on the intermediate mounting block (17) in such a manner that it can be rotated about a second axis (15), wherein, furthermore, the adjustment element (12) is mounted on the mounting block (11), in particular, via the intermediate mounting block (17), so that it can be pivoted in the manner of a rocker, and can be rotated about a first axis (14) by means of a first adjusting screw (23) supported, on the one hand, on the mounting block (11) and, on the other hand, on the adjustment element (12) or an intermediate mounting block (17) preferably against the action of a resilient element, in particular, a helical compression spring (16).

2. Device according to claim 1,
**characterised in that**
the retaining surface (10) or respectively the associated adjustment element (12) can be rotated about an axis approximately parallel to the longitudinal axis of the bone.

3. Device according to claim 1 or 2,
**characterised in that**
the mounting block (11) provides several through boreholes (13, 29) for bone screws or bone-fixing pins.

4. Device according to any one of claims 1 to 3,
**characterised in that**,
together with the adjustment element (12), the mounting block (11) can be fixed to the bone in such a manner that the adjustment element (12) is mounted in such a manner that it can be rotated about a first axis (14) extending perpendicular to the bone, especially in a posterior/anterior direction and about a second axis (15) extending perpendicular both to this first axis (14) and also to the longitudinal axis of the bone, in particular, in the medial/lateral direction.

5. Device according to any one of claims 1 to 4,
**characterised in that**
the adjustment element (12) is held on the mounting block (11) via the intermediate mounting block (17), wherein the intermediate mounting block (17) is mounted on the mounting block (11) in such a manner that it can be rotated about an axis, in particular, the first axis (14), and the adjustment element (12) is mounted on the intermediate mounting block (17) in such a manner that it can be rotated about another axis, especially the second axis (15) extending perpendicular to the first axis (14).

6. Device according to any one of claims 1 to 5,
**characterised in that**,
together with the retaining surface (10), the adjustment element (12) can be rotated about the second axis (15) and adjusted on the intermediate mounting block (17) by means of a second adjusting screw (18).

7. Device according to claim 6,
**characterised in that**
the second adjusting screw (18) provides two conical surfaces (19, 20) inclined towards one another, which correspond with complementary sloping surfaces (21, 22) on the intermediate mounting block (17) in such a manner that by moving the adjusting screw (18), the adjustment element (12) is rotated about the second axis (15) either in the dorsal or ventral direction and held in this position.

8. Device according to any one of claims 1 to 7,
**characterised in that**
mutually-corresponding markings (24, 25) are provided, on the one hand, on the adjustment element (12) and intermediate mounting block (17) and/or, on the other hand, on the intermediate mounting block (17) and the mounting block (11).

## Revendications

1. Dispositif de positionnement pour exécuter une coupe d'os, par exemple à l'extrémité proximale d'un tibia, avec une surface d'appui (10) susceptible d'être associée à l'os et destinée à recevoir un instrument de coupe ou de sciage, un gabarit d'orientation et/ou un instrument de mesure,
**caractérisé en ce que**
la surface d'appui (10) fait partie d'un élément d'ajustage (12), qui est maintenu selon une articulation à joint universel par le biais d'un support de palier intermédiaire (17) sur un support de palier (11) apte à être fixé à l'os, ledit support de palier intermédiaire (17) étant monté sur ledit support de palier (11) avec capacité de pivotement autour d'un premier axe (14) et l'élément d'ajustage (12) étant monté sur ledit support de palier intermédiaire (17) avec capacité de pivotement autour d'un second axe (15), ledit élément d'ajustage (12) étant en outre monté pivotant à la manière d'une bascule sur le support de palier (11), notamment par le biais du support de palier intermédiaire (17), et pouvant pivoter autour d'un premier axe (14) au moyen d'une première vis d'ajustage (23) prenant appui sur le palier support (11), d'une part, et sur l'élément d'ajustage (12) ou un support de palier intermédiaire (17), d'autre part, de préférence à l'encontre de l'action d'un élément élastique, notamment d'un ressort de pression à boudin (16).

2. Dispositif selon la revendication 1,
**caractérisé en ce que**
la surface d'appui (10) ou l'élément d'ajustage associé (12) peut pivoter autour de l'axe sensiblement parallèlement à l'axe longitudinal de l'os.

3. Dispositif selon la revendication 1 ou la revendication 2,
**caractérisé en ce que**
le support de palier (11) présente une pluralité de perçages (13, 29) pour le passage de vis à os ou de tiges de fixation pour os.

4. Dispositif selon l'une quelconque des revendications 1 à 3,
**caractérisé en ce que**
le support de palier (11) avec l'élément d'ajustage (12) peut être fixé à l'os de manière à ce que l'élément d'ajustage (12) soit monté avec capacité de pivotement autour d'un premier axe (14) s'étendant perpendiculairement à l'os, notamment dans la direction postéro-antérieure, ainsi qu'autour d'un second axe (15) s'étendant perpendiculairement à la fois à ce premier axe (14) et à l'axe longitudinal de l'os, notamment dans la direction médio-latérale.

5. Dispositif selon l'une quelconque des revendications 1 à 4,
**caractérisé en ce que**
l'élément d'ajustage (12) est maintenu sur le support de palier (11) par le biais du support de palier intermédiaire (17), ledit support de palier intermédiaire (17) étant maintenu sur le support de palier (11) avec capacité de pivotement autour d'un axe, notamment le premier axe (14) et l'élément d'ajustage (12) étant maintenu sur le support de palier intermédiaire (17) avec capacité de pivotement autour d'un autre axe, notamment le second axe (15), qui s'étend perpendiculairement au premier axe (14).

6. Dispositif selon l'une quelconque des revendications 1 à 5,
**caractérisé en ce que**
l'élément d'ajustage (12) avec la surface d'appui (10) peut être ajusté sur le support de palier intermédiaire (17) et entraîné en rotation autour du second axe (15) au moyen d'une seconde vis d'ajustage (18).

7. Dispositif selon la revendication 6,
**caractérisé en ce que**
la seconde vis d'ajustage (18) présente deux surfaces coniques (19, 20) inclinées en sens contraires et correspondant à des surfaces obliques complémentaires (21, 22) que présente le support de palier intermédiaire (17) de telle manière que lorsque l'on agit sur la vis d'ajustage (18), l'élément d'ajustage (12) pivote autour du second axe (15) soit en direction dorsale, soit en direction ventrale, et est maintenu dans cette position.

8. Dispositif selon l'une quelconque des revendications 1 à 7,
**caractérisé en ce que**
des repères (24, 25) sont prévus sur l'élément d'ajustage (12) et le support de palier intermédiaire (17) d'une part et/ou sur le support de palier intermédiaire (17) et le support de palier (11) d'autre part, lesdits repères correspondant entre eux.
